# DEMANDE DE BREVET EUROPEEN

(11) **EP 3 895 651 A1**
(43) Date de publication de la demande: **20.10.2021**
(21) Numéro de dépôt: 21168919.5
(22) Date de dépôt: 16.04.2021
(51) Int. Cl.: A61B 46/00, B32B 27/18, B32B 27/30, B32B 27/40, C08J 5/18, C08K 3/015, C08K 5/00, C08L 27/06, C08L 75/04

(54) **FILM ANTIMICROBIEN THERMOPLASTIQUE ÉLASTOMÈRE, COMPOSITION ET PROCÉDÉ DE FABRICATION POUR SON OBTENTION**

(30) Priorité: 17.04.2020 FR 2003916; 02.02.2021 FR 2101007
(71) Demandeur: Hexis, 34110 Frontignan (FR)
(72) Inventeur: MATEU, Michel, 34540 BALARUC-LE-VIEUX (FR)
(74) Mandataire: Rhein, Alain

(57) **Abrégé**

L'invention concerne un film (1) antimicrobien thermoplastique élastomère en polychlorure de vinyle ou en polyuréthane ou dans un mélange de ces deux matériaux.

Selon l'invention, le film est coulé dans sa masse et intègre un biocide, le biocide étant réparti directement dans la masse du film antimicrobien, caractérisé en ce qu'il comporte au moins deux couches (2 ,3, 6) superposées et thermo-soudées d'un matériau thermoplastique élastomère, au moins une des deux couches (2, 3) comporte le biocide selon une teneur massique déterminée par rapport à la masse de la couche (2, 3) qui intègre le biocide dans sa masse.

Le film (1) antimicrobien possède une application plus particulière de protection médicale.

L'invention porte également sur une composition et un procédé de fabrication qui permettent l'obtention du film (1) antimicrobien.

## Description

[La présente invention entre dans le domaine des matériaux polymériques et plus particulièrement des films thermoplastiques. L'invention s'inscrit plus précisément dans le domaine des films thermoplastiques antimicrobiens.

Dans ce contexte, l'invention vise à fournir un film thermoplastique élastomère qui peut être utilisé comme protection antimicrobienne notamment dans un contexte médical, chirurgical, vétérinaire, ou plus généralement dans le cadre de l'hygiène et la sécurité sanitaire.

En effet, il existe un besoin de protéger tant les patients que les soignant des infections associées aux soins. En ce qui concerne, le patient, une infection est dite associée aux soins lorsqu'elle survient au cours de la prise en charge du patient par un professionnel de santé. Cette infection peut intervenir tant en début de la prise en charge pendant la phase de diagnostic que lors de la prise en charge elle-même : thérapie, soins palliatifs, soins préventifs, soins opératoires ou post-opératoires. Au sein du milieu hospitalier, ces infections sont dites « nosocomiales ».

Ces infections peuvent être liés à un germe bactérien issu par exemple de la famille des Staphylocoques, des Salmonelles, des Listeria etc. Ces infections peuvent être problématiques lorsque le germe infectieux est résistant ou multirésistant aux antibiotiques. Le plus connu de ces germes résistants est le Staphylocoque doré ou *Staphylococcus aureus.* Ce type d'infection peut entrainer des complications graves qui dans des cas extrêmes sont susceptibles de mener à l'amputation ou au décès du patient.

Cependant, les virus peuvent également être source d'infections liées aux soins et plus particulièrement les virus respiratoires tels que les virus de la grippe, le virus respiratoire syncytial ou encore les coronavirus. Dans les vingt premières années du XXIème siècle, cette dernière famille est à l'origine de trois épidémies mortelles : une première épidémie s'est répandue en 2003, elle était liée au coronavirus SRAS-CoV, une deuxième épidémie s'est développée en 2012 et était due au coronavirus MERS-CoV en 2012, enfin récemment une troisième épidémie s'est déclarée fin 2019, début 2020 suite à la propagation du coronavirus SARS-CoV-2 autrement appelé Covid-19. Cette dernière a été déclarée officiellement comme une pandémie le 12 mars 2020 par l'OMS (Organisation Mondiale de la Santé).

Cette crise sanitaire planétaire a mis, notamment, en lumière le manque de protection du personnel soignant vis-à-vis de ce virus respiratoire hautement contagieux. Ainsi, de nombreux soignants ont été contaminés par le Covid-19 alors qu'ils prodiguaient des soins aux patients eux-mêmes infectés.

Les champignons ou mycoses sont également un autre type de germes qui sont susceptibles d'être une source d'infections nosocomiales. A titre informatif, les champignons de la famille des Aspergillus ou des Candidas sont les plus couramment identifiés comme source d'infections associées aux soins.

Les infections associées aux soins sont problématiques à bien des égards, tout d'abord leur nombre important. En temps normal, chaque année un pays comme la France déplore environ 1 000 000 de patients victimes d'une infection nosocomiale dont la gravité est variable. Cependant, parmi ce recensement, environ 10 000 patients décèdent des suites d'une infection associée à des soins.

D'un point de vue sanitaire, il est difficile d'accepter qu'un patient puisse être infecté au cours de son parcours de soins par un germe qui n'est pas relation directe avec la pathologie qui l'a conduit à suivre des soins. De plus, une telle infection a bien souvent pour conséquence la prolongation du parcours de soins du patient. Outre les préjudices moraux et physiques subits par certains patients qu'il n'est aucunement question d'occulter, ces infections associées aux soins génèrent des dépenses supplémentaires tant en termes de la prise en charge par le personnel de santé, que d'un point de vue du matériel et des médicaments. Dans un pays comme la France, ces dépenses sont prises en charge par le système de remboursement des soins, or ce n'est pas le cas dans la plupart des pays.

Les modes de transmission de ces infections sont variés, ils peuvent être aériens dans le cas de la tuberculose, des bactéries de la famille des Légionelles ou des virus comme le SARS Cov-2, par un produit contaminé comme les dérivés de sang, du matériel médical contaminé tel qu'un fibroscope etc. Toutefois, il est estimé que 80% des infections associées aux soins sont manuportées, c'est-à-dire, transmises par les mains.

A n'en pas douter, la prévention de tous les désagréments humains et financiers que nous venons d'exposer constitue un des enjeux majeurs du système de soins de nos sociétés. Or, un des objectifs de la présente invention consiste à réduire le risque de transmission des infections associées aux soins tant pour les patients que le personnel soignant.

Des solutions de protection des patients ou du personnel soignant existent déjà, ainsi les documents EP 2 229 193, EP 0 136 900, EP 2 908 635, décrivent un drap médical opaque qui comprend un support textile imprégné d'une préparation antimicrobienne. La préparation antimicrobienne peut comprendre un agent antimicrobien à base de guanidine ou de bronopol. Le support textile est constitué de mailles tissées qui comportent des trous, par conséquent, le drap médical obtenu par imprégnation possède une surface non plane qui comporte des creux et/ou des recoins. Ces creux et recoins favorisent l'accumulation de microorganismes. De fait, l'efficacité de ce type de drap médicaux n'est pas optimale et pourraient même s'avérer à contre-emploi en cas d'une trop grande accumulation de microorganismes à la surface du textile imprégné.

Le brevet EP 0 721 024 décrit quant à lui un film tri-couche opaque dans lequel un corps central est assemblé inférieurement et supérieurement par deux couches externes. Le corps central est discontinu et constitué billes de biocide et d'adhésifs. Une première couche externe poreuse qui est perméable au gaz et/ou à l'eau, elle est constituée par un textile tissé en fibres naturelles ou synthétiques. La deuxième couche externe est imperméable à l'air et à l'eau. Ces différentes couches sont assemblées les unes avec les autres par lamination. Ce document propose un film multicouche complexe dont les couches externes ne possèdent pas de propriétés antimicrobiennes. Les microorganismes sont piégés, en passant au travers de la couche poreuse, dans le corps central qui contient le biocide. Ce film multicouche ne permet pas de détruire les microorganismes qui se déposent sur ses deux faces. Par ailleurs, la structure complexe de ce film multicouche le rend onéreux à produire.

Dans ce contexte, la demanderesse a développé une solution technique capable de fournir un film antimicrobien efficace biologiquement et dont les contraintes de fabrication sont restreintes en vue d'accélérer les cadences de production et de réduire les coûts de fabrication.

L'invention a trait à un film antimicrobien thermoplastique élastomère en polychlorure de vinyle ou en polyuréthane ou un mélange de ces matériaux, le film antimicrobien étant coulé dans sa masse et intégrant le biocide étant réparti directement dans la masse.

Le film antimicrobien se caractérise en ce qu'il comporte au moins deux couches superposées et thermo-soudées d'un matériau thermoplastique élastomère, au moins une des deux couches comporte le biocide.

Le film antimicrobien est un dispositif de protection médicale. Il est peut-être utilisé comme drap médical, film de protection médical ou encore comme une housse de protection antibactérienne.

Avantageusement, le film antimicrobien est étanche à l'air et à l'eau, il peut ainsi former une barrière de protection contre les micro-organismes.

Selon un premier exemple de réalisation de l'invention, les deux couches intègrent un biocide dans leur masse.

Selon un second exemple de réalisation de l'invention, le film antimicrobien comporte au moins trois couches superposées et thermo-soudées d'un matériau thermoplastique élastomère, le film comportant un corps central recouvert supérieurement et inférieurement par deux couches externes, au moins l'une des deux couches externes intègre un biocide dans sa masse, de préférence, les deux couches externes intègrent un biocide dans leur masse.

Selon l'invention, la teneur massique en biocide est comprise entre 0,1 et 30 % en masse par rapport à la masse totale d'une couche qui intègre le biocide, de préférence, la teneur massique en biocide est supérieure 10 % et inférieure à 30% en masse par rapport à la masse totale d'une couche qui intègre le biocide. Encore de préférence, la teneur massique en biocide est comprise entre 15 % et 25% en masse par rapport à la masse totale d'une couche qui intègre le biocide.

Le film antimicrobien possède en outre une épaisseur comprise entre 50 µm à 300 µm et de préférence le film antimicrobien possède une épaisseur comprise entre 100 µm à 200 µm.

Avantageusement, et à l'inverse des draps médicaux de l'état de la technique, le film antimicrobien possède une transparence d'au moins 70 % à la lumière visible, de préférence, il possède une transparence d'au moins 90 % à la lumière visible. Cette caractéristique permet au soignant d'exécuter des gestes médicaux alors que le film antimicrobien est utilisé comme drap médical.

Selon l'invention le film antimicrobien comporte un allongement à la rupture d'au moins 150 %, de préférence il possède un allongement à la rupture d'au moins 180%. Le film antimicrobien possède également une résistance à la traction d'au moins 2 kN/m, de préférence, la résistance à la traction est d'au moins 4kN/m. De telles propriétés mécaniques contribuent à réduire le risque de déchirures du film antibactérien. Le film constitue alors une barrière de protection fiable.

De plus, le film antimicrobien est dermo-compatible, il peut ainsi être utilisé comme protection médicale. En particulier, le film antimicrobien est particulièrement adapté pour l'utilisation comme un drap médical ou comme une protection de matériel médical contre la transmission de micro-organismes infectieux.

La présente invention concerne également une composition polymérique de préparation d'un film antimicrobien selon l'invention.

Cette composition se caractérise en ce qu'elle comprend en pourcentages massiques par rapport à la masse totale :
∘ 1 à 6 % d'un biocide,
∘ 25 à 70 % d'au moins un solvant,
∘ 25 à 70 % d'au moins un précurseur de polyuréthane ou d'au moins un précurseur de polychlorure de vinyle,
∘ 1 à 5 % d'au moins un agent de contrôle.

En vue de coulé le film selon un procédé d'enduction, le biocide choisi est un composé aprotique stable thermiquement à des températures supérieures à 100°C, de préférence, le biocide est un composé stable thermiquement à des températures supérieures à 150°C. Préférentiellement, le biocide est un sel d'argent ou un composé de type isothiazole.

En vue une solution homogène de précurseurs de polymère, le solvant comporte un solvant dispersant choisi parmi les composés suivants :
∘ un solvant polaire aprotique de type acétate choisi parmi les solvants suivants : l'acétate de méthyle, acétate d'éthyle, acétate de propyle, acétate de butyle, acétate de pentyle, acétate d'hexyle, acétate d'heptyle, acétate d'octyle ou un mélange de ces composés ; ou
∘ un solvant polaire aprotique de type cétonique et choisi parmi les solvants suivants : di-méthyl-cétone, éthyl-méthyl-cétone, propyl-méthyl-cétone, butyl-méthyl-cétone, méthyl-butyl-cétone, méthyl-isobutyl-cetone, méthyl-pentyl-cétone, di-éthyl-cétone, éthyl-propyl-cétone, éthyl-butyl-cétone, éthyl-pentyl-cétone, di-propyl-cétone, butyl-propyl-cétone, pentyl-propyl-cétone, di-butyl-cétone, butyl-pentyl-cétone, ou un mélange de ces composés.

Selon une variante de la composition polymérique de l'invention, la composition comprend en pourcentages massiques par rapport à la masse totale :
∘ 1 à 6% de biocide ;
∘ 25 % à 45% comprend deux précurseurs de polychlorure de vinyle : une résine de chlorure de vinyle et un plastifiant polymérique, le ratio massique de chlorure de vinyle étant 1 :3 par rapport au plastifiant polymérique.
∘ 45 % à 70 % d'au moins un solvant ;
∘ 1 à 5 % d'un agent de contrôle constitué par un stabilisant thermique de type sel de baryum.

Cette variante permet de fabriquer un film antimicrobien en polychlorure de vinyle conforme à l'invention. A cet effet, on utilise une résine de chlorure de vinyle de type homopolymère dont la masse moléculaire moyenne est comprise entre 150 000 g/mol et 250 000 g/mol. En parallèle, le plastifiant polymérique peut être un polymère de type polyadipate ou polysebaçate.

Selon l'invention, le solvant peut comporter un mélange d'un solvant dispersant et d'un solvant diluant, le solvant diluant est également composé d'un mélange d'au moins un composé de nature aromatique avec au moins un composé de nature aliphatique selon un ratio 4 :1, le solvant diluant est choisi parmi les composés suivants :
∘ Un solvant d'hydrocarbure aromatique aprotique possédant 7 à 10 carbones choisi parmi la liste des composés suivants : toluène, xylène, m-xylène, p-xylène, éthylbenzène, mésitylène, durène, ou un mélange de ces composés ;
∘ Un solvant d'hydrocarbures aliphatiques dont la formule chimique possède entre 6 et 13 carbones ou un mélange de ces hydrocarbures aliphatiques.

Une autre variante de la composition permet de fabriquer un film antimicrobien en polyuréthane. Selon cette variante la composition polymérique comprend en pourcentages massiques par rapport à la masse totale :
∘ 1 à 6% de biocide ;
∘ 10% à 35% d'un premier précurseur de polyuréthane comprenant un mélange d'un composé polyol à courtes chaines et d'un composé polyol à longues chaines selon un ratio du composé polyol à courtes chaines 1 :4 par rapport au composé polyol à longue chaines ;
∘ 15 % à 35% d'un second précurseur de polyuréthane comportant au moins un composé poly-isocyanate ;
∘ 25 % et 50 % d'au moins un solvant ; et
∘ 1 à 5 % d'un agent de contrôle comprenant un inhibiteur de polymérisation.

Selon cette variante, l'agent de contrôle comportant en outre un catalyseur de polymérisation qui est chelaté par l'inhibiteur de polymérisation.

Par ailleurs, l'invention se rapporte également au procédé de fabrication d'un film antimicrobien conforme de l'invention. Le procédé se caractérise en ce qu'il comprend :
∘ une étape de préparation d'une composition polymérique conforme de l'invention qui intègre un biocide, la composition étant mélangée jusqu'à obtention d'une solution à l'état liquide homogène ;
∘ une étape de formation d'une première couche du film à l'état cohésif qui comporte :
   - une opération d'enduction de ladite composition polymérique à l'état liquide sur un support solide,
   - une opération de séchage et/ou de polymérisation de ladite composition polymérique enduite sur le support solide afin d'obtenir une première couche polymérique à l'état cohésif ;
∘ une étape de formation d'une deuxième couche du film à l'état cohésif qui comporte :
   - une opération d'enduction de ladite composition polymérique sur une couche polymérique à l'état cohésif reposant directement ou indirectement sur le support, et
   - une opération de séchage et/ou de polymérisation de ladite composition polymérique enduite à l'état liquide afin d'obtenir une deuxième couche polymérique à l'état cohésif permettant d'obtenir un film antimicrobien monobloc comprenant, d'une part, deux couches intégrant un biocide, et d'autre part, une épaisseur comprise entre 50 µm et 300 µm,.

Afin de réaliser un film antimicrobien à au moins trois couches, le procédé de fabrication peut comprendre une étape de formation d'un corps central du film qui est constitué par au moins une couche polymérique, l'étape de formation du corps central comportant :
- Une opération de préparation d'une deuxième composition polymérique incluant un matériau thermoplastique élastomère ou des précurseurs de ce matériaux, la deuxième composition étant mélangée jusqu'à obtention d'un solution homogène et transparente à l'état liquide,
- Une opération d'enduction de la deuxième composition polymérique sur la première couche à l'état cohésif,
- Une opération de séchage et/ou de polymérisation de la deuxième composition polymérique afin de la faire passer de l'état liquide à l'état cohésif formant ainsi une couche polymérique constituant le corps central solidaire de la couche à l'état cohésif sur laquelle il a été enduit.

Selon l'invention, l'étape de formation du corps central est intercalée entre les étapes de formation de la première et de la deuxième couche comportant du biocide.

Les opérations de séchage qui sont effectuées dans le cadre du procédé de fabrication de l'invention, sont opérées par application d'une température comprise entre 70°C et 200°C pendant une durée inférieure à 10 minutes.

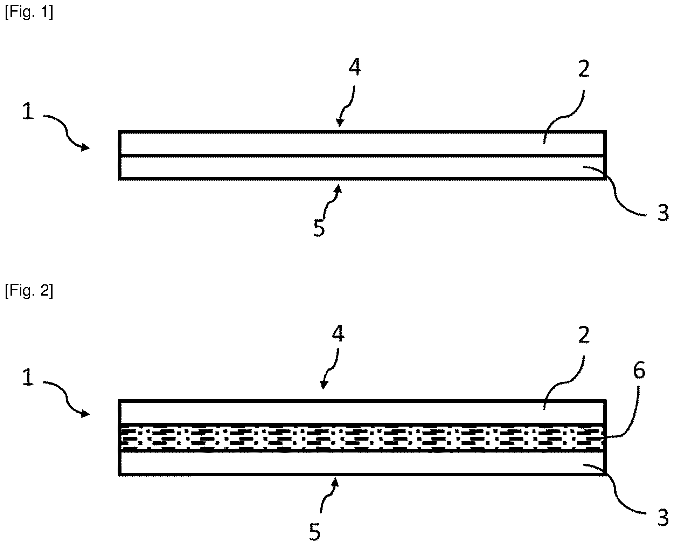

D'autres particularités et avantages apparaitront dans la description détaillée qui suit, de deux exemples de réalisation, non limitatifs, de l'invention illustrés par les figures 1 et 2 placées en annexe et dans lesquelles :
[Fig. 1] est une représentation d'une coupe transversale d'un film thermoplastique élastomère conforme à un premier exemple de réalisation de l'invention.
[Fig. 2] est une représentation d'une coupe transversale d'un film thermoplastique élastomère conforme à un deuxième exemple de réalisation de l'invention.

L'invention concerne un film 1 antimicrobien possédant des propriétés antimicrobiennes permanentes. Le film 1 antimicrobien est un film thermoplastique élastomère. Les propriétés antimicrobiennes du film 1 sont particulièrement intéressantes dans le cadre d'activités dans lesquelles il y a un fort risque de transmission d'une infection liée aux soins. L'utilisation du film 1 antimicrobien comme un drap médical et/ou chirurgical s'avère particulièrement efficace pour réduire les risques de transmission de microorganismes infectieux tant pour le patient que pour le personnel soignant. Par exemple, dans le cas d'une hospitalisation, le film antimicrobien 1 peut être utilisé comme une couverture ou un drap pour recouvrir le patient. Le film antimicrobien 1 peut être appliquée directement sur le patient, ou positionné tel un tunnel au-dessus du patient à l'aide d'un support fixé au lit ou au brancard sur lequel le patient se tient. Il est également possible d'utiliser ce film dans le cadre d'un environnement médical dédié aux patients immunodéprimés.

Dans ce contexte, le film 1 antimicrobien constitue alors une barrière physique entre le patient et l'environnement qui l'entoure protégeant ainsi le patient d'une infection manuportée notamment. En outre, le film 1 antibactérien utilisé comme tel constitue également une barrière physique entre le patient et le personnel soignant, protégeant aussi le personnel soignant d'une infection manuportée depuis le patient.

De surcroît, les propriétés antimicrobiennes du film 1 permettent d'éliminer les germes qui pourraient entrer en contact avec le film thermoplastique 1.

Le film 1 antimicrobien est à usage unique. Le film selon l'invention répond à la norme établit par l'article 1, §1.1 de l'annexe IX de la Directive Européenne 93/42/CEE. A ce titre, le film 1 antimicrobien peut être utilisé en continu pour un même patient pendant une période maximale de trente jours successifs.

Le film 1 antimicrobien est réalisé en thermoplastique élastomère. Le matériau thermoplastique élastomère peut être choisi parmi la liste des matériaux suivants : le polychlorure de vinyle (PVC), le polyuréthane (PU) ou un mélange de ces matériaux.

Selon l'invention, le matériau thermoplastique élastomère possède un pourcentage massique compris entre 50 à 99,9 % par rapport à la masse totale du film 1 antimicrobien.

En particulier, le film 1 antimicrobien est coulé dans sa masse. Ce qui contribue à produire un film 1 plein et homogène sans inclusion d'air ou de fibre le rendant « imperméable » à l'air et à l'eau. A l'inverse, d'un textile tissé, l'imperméabilité à l'air du film 1 selon l'invention permet de fournir une barrière bilatérale de protection fiable vis-à-vis des microorganismes et notamment des virus dont la taille varie entre 10 nm pour les plus petits et jusqu'à 400 nm pour les plus grands.

Par ailleurs, le film 1 antimicrobien possède une épaisseur comprise entre 50 µm et 300 µm. De préférence, le film 1 antimicrobien possède une épaisseur comprise entre 100 µm à 200 µm.

L'utilisation du film 1 antimicrobien comme drap médical requiert une épaisseur de film comprise entre 100 µm et 300 µm. Cependant, un film 1 qui possède une épaisseur comprise entre 50 µm et 100 µm peut être utilisé comme film de protection du matériel électronique en milieu médical comme un moniteur, un clavier, un écran tactile ou traditionnel, des téléphones portables etc. En pratique, le film 1 antimicrobien est utilisé comme housse de protection jetable. L'objet à protéger est enveloppé par un morceau de film 1 qui est ensuite fermé par thermo soudage ou un soudage haute fréquence ou ultrason.

La nature du ou des matériau(x) thermoplastique(s) utilisé(s) et l'épaisseur du film 1 antimicrobien permet d'obtenir des propriétés spécifiques. En particulier, les caractéristiques d'épaisseur et les matériaux choisis permettent d'obtenir un film 1 antimicrobien qui possède des propriétés de transparence à la lumière du visible. Comme cela est connue de l'homme du métier, la lumière du visible concerne les ondes électromagnétiques de longueurs d'onde comprises entre 400 nm et 800 nm. Pour rappel, la transparence désigne la capacité d'un matériau à ne pas interagir avec une onde. Selon l'invention, le film 1 possède une transparence d'au moins 70 % à la lumière visible, de préférence, le film 1 possède une transparence d'au moins 90 % à la lumière visible. Avantageusement, le caractère translucide du film 1 antimicrobien tel que défini, assure la visibilité du patient et des gestes médicaux et/ou chirurgicaux facilitant ainsi les soins.

. L'élasticité est une autre propriété physique que peut posséder le film 1 antimicrobien. Cette propriété est également liée à la nature du matériau et à l'épaisseur du film 1 antibactérien. Avantageusement, le caractère élastique permet de prévenir tout déchirement du film 1 et donc toute rupture de la barrière de protection du patient et/ou du personnel soignant. Cela est particulièrement utile lorsque le personnel soignant exécute des gestes médicaux et/ou chirurgicaux pour traiter le patient recouvert du film 1 antimicrobien.

Selon l'invention, le film 1 antimicrobien comporte un allongement à la rupture d'au moins 150 %. De préférence, le film 1 antimicrobien possède un allongement à la rupture d'au moins 180%.

La résistance à la traction est également une propriété permettant de réduire le risque de déchirure du film 1 antimicrobien. Selon l'invention, le film 1 antimicrobien possède une résistance à la traction d'au moins 2 kN/m, de préférence, la résistance à la traction du film 1 antimicrobien est d'au moins 4 kN/m.

Les mesures d'allongement à la rupture et de résistance à la traction ont été opérées selon une méthode similaire à la méthode NF EN 14410 en utilisant un dynamomètre de type « MTS criterion model 42 ».

Selon l'invention, le film 1 antimicrobien intègre un biocide directement dans la masse du film 1. Le biocide est ainsi réparti de façon homogène et uniforme dans la masse du film 1. Le biocide choisi est actif tant pour les germes bactériens, viraux ou encore contre les champignons.

En particulier, le biocide choisi est actif selon la norme ISO 22196 sur les souches bactériennes suivantes Staphylocoque doré (*Staphylococcus aureus*), Staphylococcus aureus résistant à la méticilline (SARM), Salmonelle (*Salmonelle enterica*)*,* Listeria (*Listeria monocytogenes*)*, Escherichia coli* et *Pseudomonas aeruginosa.*

A ces fins, le biocide peut comprendre des nanoparticules d'argent ou un composé de la famille des isothiazoles. A titre indicatif, les nanoparticules d'argent peuvent être complexées sous forme de sels d'argent. Ces biocides sont conformes à l'article 95(1) du règlement sur les produits biocides correspondant à la Directive Européenne 98/8/EC. L'Agence Européenne de la Chimie (ECHA) tient à jour une liste des produits qui répondent à ces normes. En ce sens, d'autres biocides conforme à cette réglementation peuvent être utilisés dans le cadre de l'invention. Ceci dans la mesure où ces biocides sont conformes aux critères de sélection de l'invention (voir la description de la composition ci-après).

Le film 1 antimicrobien tient ses propriétés antimicrobiennes du biocide intégré dans sa masse. Plus particulièrement, le pourcentage massique du biocide est compris entre 0,1 à 6% de la masse totale du film 1 antimicrobien. De préférence, le pourcentage massique de biocide par rapport à la masse totale du film est inférieur 0,6%. Encore de préférence, le pourcentage massique de biocide par rapport à la masse totale du film est inférieur 0,3 %.

Avantageusement, selon cette intégration de biocide choisi selon la norme ISO 22196 permet d'obtenir, à la surface du film 1 antibactérien, une réduction logarithmique des colonies bactériennes comprise entre log 2 et log 5 soient une réduction des colonies bactériennes entre 99% et 99,999% à la surface du film 1 antimicrobien.

Comme illustré aux figures 1 et 2, le film 1 antimicrobien comprend au moins deux couches 2, 3 superposées et solidaires entre elles. Les deux couches 2, 3 sont thermo-soudées. Ceci permet d'éviter d'utiliser une substance adhésive pour maintenir la cohésion du film. Parmi ces deux couches 2, 3, au moins l'une d'entre elles possède un biocide intégré dans sa masse. Dans cet exemple, le biocide est réparti de façon homogène et uniforme dans la masse de chaque couche 2, 3 qui comporte du biocide.

Un premier exemple de réalisation est illustré à la figure 1. Dans cet exemple, le film comporte deux couches 2, 3 superposées qui possèdent chacune un biocide intégré dans la masse du matériau polymérique qui les compose. Les surfaces extérieures des deux couches 2 ,3 définissent respectivement une face 4, 5 du film 1 antimicrobien.

Il est à noter que les deux couches 2, 3 superposées peuvent posséder la même composition ou bien une composition différente. Cela peut être utile pour conférer des propriétés particulières à une des faces du film 1 antimicrobien.

La figure 2 illustre un deuxième exemple de réalisation de l'invention. Selon cet exemple, le film 1 antimicrobien comporte une troisième couche 6. La troisième couche 6 est intercalée entre les autres couches 2, 3. Selon cet exemple, le film 1 antimicrobien comporte trois couches 2, 3, 6 successives superposées.

La troisième couche 6 est ainsi considérée comme le corps central du film 1 antimicrobien. Le corps central recouvert supérieurement et inférieurement par deux couches 2, 3 externes.

La nature du corps central peut varier afin d'obtenir des propriétés physique, mécanique, optiques ou d'élasticité spécifiques. Cependant, dans tous les cas le corps central est réalisé dans un matériau thermoplastique élastomère qui est obtenu par coulage sur une couche 2, 3 préformée.

Selon cet exemple de réalisation, au moins une des deux couches externes intègre un biocide dans sa masse. Ici, les deux couches 2, 3 externes intègrent un biocide dans leur masse. Dans cet exemple, le biocide est réparti de façon homogène et uniforme dans la masse de chaque couche externe 2, 3 qui comporte du biocide.

De manière générale, comme pour le premier exemple de réalisation, les trois couches 2, 3 ,6 sont thermocollées entre elles.

Les deux exemples de réalisation illustrés aux figures 2 et 3 peuvent être employés indifféremment. Cependant, l'exemple illustré à la figure 2 permet, au travers du corps central, de conférer des propriétés spécifiques au film 1 tout en conservant deux faces 4, 5 biologiquement actives contre les microorganismes.

Conformément à l'invention, les deux exemples de réalisation du film 1 antimicrobien peuvent posséder les propriétés de transparence, d'élasticité et de résistance à la traction. En sus, le film 1 antimicrobien est préférentiellement dermocompatible.

Des tests dermatologiques ont été menés sur un échantillon de quinze individus adultes âgés de 18 à 65 ans. Ces tests consistaient à appliquer un spécimen de film 1 antibactérien 4 cm² sur la peau de chaque individu pendant une durée de 6 heures consécutives. Un premier contrôle visuel de la peau au niveau de l'emplacement du spécimen a été opéré lors du retrait du spécimen, un deuxième contrôle a été effectué 30 minutes après le retrait du spécimen. Enfin un troisième et dernier contrôle a été opéré 24 heures après le retrait du spécimen. Pour l'ensemble des quinze individus qui ont participé, aucun signe d'irritation n'a été observé lors des trois contrôles.

Le tableau 1 ci-dessous illustre plusieurs films 1 antimicrobiens conformes à l'invention.

**[Tableau 1]**

| Exemples de film antimicrobien | Nombre de couches | Epaisseur du film en µm | Epaisseur couches chargées en biocide en µm | Epaisseur corps central en µm | Application du film |
|---|---|---|---|---|---|
| A | 2 | 60 à 80 | 30 à 40 | / | Protection dispositif médicaux |
| B | 2 | 100 à 140 | 50 à 70 | / | Drap médical |
| C | 3 | 100 à 200 | 30 à 50 | 40 à 100 | Drap médical |
| D | 3 | 200 à 300 | 50 à 75 | 150 à 200 | Drap médical |

L'exemple A décrit un film 1 antibactérien comportant deux couches 2, 3 chargées en biocide, l'épaisseur totale de ce film est inférieure à 90 µm, un tel film peut être utilisé comme film de protection du matériel dans un milieu médical tel que décrit précédemment. De préférence, ce type de film est réalisé en PVC.

A l'inverse les exemples B, C et D sont des films 1 antibactériens à deux couches 2, 3 (exemple B) ou trois couches 2, 3, 6 (exemples C et D) dont l'épaisseur totale est supérieure ou égale à 100 µm. Ces trois films sont plus particulièrement utilisés comme drap tel que décrit précédemment.

Comme évoqué précédemment, le film 1 antimicrobien est obtenue par au moins deux enductions d'une composition polymérique de préparation du film.

De fait l'invention concerne également une composition polymérique pour la préparation du film 1 antimicrobien. Cette composition polymérique selon l'invention comprend en pourcentages massiques par rapport à la masse totale :
∘ 1 à 6 % d'un biocide,
∘ 25 à 70 % d'un au moins solvant,
∘ 25 à 70 % d'au moins un précurseur de polyuréthane ou d'au moins un précurseur de polychlorure de vinyle, et
∘ 1 à 5 % d'au moins un agent de contrôle.

La composition polymérique selon l'invention consiste en pourcentages massiques par rapport à la masse totale en:
∘ 1 à 6 % d'un biocide,
∘ 25 à 70 % d'au moins un solvant,
∘ 25 à 70 % d'au moins un précurseur de polyuréthane ou d'au moins un précurseur de polychlorure de vinyle,
∘ 1 à 5 % d'au moins un agent de contrôle.

La composition polymérique intègre un biocide afin de conférer au film 1 antibactérien ses propriétés antimicrobiennes. De préférence, le biocide est un composé aprotique stable thermiquement à des températures supérieures à 100°C, de préférence, le biocide est un composé stable thermiquement à des températures supérieures à 150°C. Ceci permet d'enduire la composition sur un support et d'évaporer le solvant afin d'obtenir une couche 2, 3 du film 1 antimicrobien.

Le biocide selon l'invention peut comprendre des nanoparticules d'argent. De préférence, les nanoparticules d'argent sont complexées sous forme de sels d'argent. A titre, indicatif le biocide peut être choisi parmi la liste des sels d'argent suivants : nitrate d'argent, les silicates d'argent, le thiosulfate d'argent, ou un mélange de ces composés.

Les nanoparticules d'argent peuvent également être supportées par des complexes zéolites d'argent et de zinc.

Les composés de type isothiazole sont également des biocides stables aux températures précédemment exposées. Le biocide peut ainsi comporter en combinaison avec des nanoparticules d'argent ou des sels d'argents des composés de type isothiazole. Selon l'invention le biocide peut comporter un composé de type isothiazole choisi parmi la liste suivantes : 2-butyl-benzo[d]isothiazol-3-one (BBIT), 2-methyl-1,2-benzothiazol-3(2H)-one (MBIT), 2-methyl-2H-isothiazol-3-one (MIT), 2-octyl-2H-isothiazol-3-one (OIT), 5-Chloro-2-methyl-2H-isothiazol-3-one (CIT), 5-chloro-2-methyl-2H-isothiazol-3-one (CMIT) ou un mélange de ces composés.

La composition polymérique selon l'invention comporte un solvant comprenant au moins un solvant dispersant. Dans cet exemple, il est préférable d'utiliser un solvant polaire aprotique qui permettent d'optimiser la solvatation des précurseurs de polymère intégrés à la composition. Les solvants de la composition polymérique sont importants, la composition doit se trouver à l'état liquide pour permettre une opération d'enduction. A cet effet, la viscosité de la composition doit être comprise entre 200 et 10 000 mPa/s.

Leur caractère aprotique permet notamment d'éviter qu'un précurseur du polyuréthane tel qu'un composé isocyanate ne réagisse avec le solvant.

Selon l'invention le solvant polaire aprotique peut être de type acétate et choisi parmi les solvants suivants : l'acétate de méthyle, acétate d'éthyle, acétate de propyle, acétate de butyle, acétate de pentyle, acétate d'hexyle, acétate d'heptyle, acétate d'octyle ou un mélange de ces composés.

De manière alternative ou en combinaison, le solvant polaire aprotique peut être de type cétonique et choisi parmi les solvants suivants : di-méthyl-cétone, éthyl-méthyl-cétone, propyl-méthyl-cétone, butyl-méthyl-cétone, méthyl-butyl-cétone, méthyl-isobutyl-cetone, méthyl-pentyl-cétone, di-éthyl-cétone, éthyl-propyl-cétone, éthyl-butyl-cétone, éthyl-pentyl-cétone, di-propyl-cétone, butyl-propyl-cétone, pentyl-propyl-cétone, di-butyl-cétone, butyl-pentyl-cétone, ou un mélange de ces composés.

Selon l'invention, il existe deux variantes de réalisation de la composition polymérique. Une première variante utilise des précurseurs de polyuréthane, alors que la seconde variante utilise des précurseurs de polychlorure de vinyle (PVC). De fait, il est possible d'obtenir un film 1 antibactérien en PVC ou en polyuréthane voir dans un mélange de ces deux matériaux.

La première variante permet d'obtenir un film 1 antibactérien en polyuréthane. Selon cette première variante, la composition polymérique comprend plus particulièrement en pourcentages massiques par rapport à la masse totale :
∘ 1 à 6% de biocide ;
∘ 10% à 35% d'un premier précurseur de polyuréthane comprenant un mélange d'un composé polyol à courtes chaines et d'un composé polyol à longues chaines selon un ratio du composé polyol à courtes chaines 1 :4 par rapport au composé polyol à longue chaines ;
∘ 15 % à 35 % d'un second précurseur de polyuréthane comportant au moins un composé poly-isocyanate ;
∘ 25 % et 50 % d'au moins un solvant ;
∘ 1 à 5 % d'un agent de contrôle comprenant un inhibiteur de polymérisation.

De préférence, la première variante de la composition polymérique consiste en pourcentages massiques par rapport à la masse totale :
∘ 1 à 6% de biocide ;
∘ 10% à 35% d'un premier précurseur de polyuréthane comprenant un mélange d'un composé polyol à courtes chaines et d'un composé polyol à longues chaines selon un ratio du composé polyol à courtes chaines 1 :4 par rapport au composé polyol à longue chaines ;
∘ 15 % à 35 % d'un second précurseur de polyuréthane comportant au moins un composé poly-isocyanate ;
∘ 25 % et 50 % d'au moins un solvant ;
∘ 1 à 5 % d'un agent de contrôle comprenant un inhibiteur de polymérisation.

La première variante de la composition comporte un mélange polyols à courtes et longues chaines qui permet de contrôler les propriétés mécaniques du film 1 antimicrobien.

Le ratio 1 :4 en polyols en courtes chaines par rapport aux polyols à longues chaines permet d'obtenir un film 1 antimicrobien qui comporte les propriétés d'élasticité décrites précédemment.

De manière connue, les composés de type polyol à courtes chaines rigidifient la structure de la couche de polyuréthane qui sera formée. A l'inverse, les composés de types polyol à longues chaines assouplissent la structure de la couche de polyuréthane qui résulte de leur polymérisation.

Selon l'invention, un composé de type polyol à courtes chaines comporte de préférence une masse molaire inférieure à 200 g/mol, et peut être notamment choisi parmi la liste de composés de type polyol aliphatique saturé suivants : 2-méthyl-propanediol, 2,2-diméthyl-propanediol, propane diol, éthane diol, 1,4-butane diol, 1,3-butanediol, 1,2-butane diol, 1,6-hexanediol, 1,5-hexanediol, 1,4-hexanediol, 1,3-hexanediol, 1,2-hexanediol, 2-méthyl hexane diol, 3-méthyl-hexanediol, 4-méthyl-hexandiol, 2,2-diméthyl hexanediol, 3,3-diméthyl-hexanediol, 4,4-diméthyl-hexandiol, 2,3-diméthyl-hexanediol, 2,4-diméthyl-hexanediol, 2,5-diméthyl hexanediol, 2,6-diméthyl-hexanediol, 3,4-diméthyl-hexanediol, 3,5-diméthyl-hexanediol, 3,6-diméthyl-hexanediol, 4,5-diméthyl hexanediol, 4,6-diméthyl-hexanediol éthylène glycol, 1,2-pentandiol, 1,3-pentandiol, 1,4-pentandiol, 1,5-pentandiol, 2,3-pentandiol, 2,4-pentandiol, diéthylène glycol, triéthylène glycol, tetraéthylène glycol, propylène glycol, dipropylène glycol, tripropylène glycol.

Parallèlement, un composé de type polyol à longues chaines comporte de préférence une masse molaire supérieure à 800 g/mol et peut être notamment choisi parmi la liste de familles de polymère suivantes : poly carbonate polyol, poly éther polyol, poly ester polyol, poly uréthane polyol, polyol vinylique, polyacrylate polyol, ainsi que les familles dérivées de plusieurs types telles que les poly ester-carbonate polyols ou encore des polyols issus de la chimie végétale tels que le glycérol qui peut être obtenu par hydrolyse des triglycérides ou issus du glucose tels que l'isosorbide ou le sorbitol.

Afin d'obtenir la formation de polyuréthane constituant une couche 2, 3 du film 1 antibactérien, la composition polymérique selon l'invention comporte également un composé de type isocyanate et plus particulièrement de type poly-isocyanate.

De manière connue, un composé de type poly-isocyanate est un précurseur de polyuréthane adapté à réagir avec une fonction alcool de manière à former un polymère présentant un motif uréthane.

Selon la première variante de la composition, le composé de type poly-isocyanate peut notamment être choisi parmi des produits commerciaux di et/ou trifonctionnel tels qu'un oligomère de 4,4'-méthylène bis(cyclohexyl isocyanate) couramment dénommé « H12MDI », un oligomère d'hexane diisocyanate couramment dénommé « HDI » ou encore un oligomère d'isophorone diisocyanate couramment dénommé « IPDI ».

Afin d'obtenir un film 1 antibactérien dermocompatible, la composition comprend un mélange de polyols et le ou les composé(s) isocyanate(s) en proportion stœchiométrique. Cela permet d'éviter que le film 1 antibactérien comporte un trop grand nombre de monomères libres qui pourraient irriter la peau du patient.

La première variante de la composition comprend un agent contrôle comprenant un inhibiteur de polymérisation. L'inhibiteur de polymérisation maintient pendant un temps la composition à l'état liquide de manière à effectuer une enduction homogène de la composition polymérique selon la première variante.

. De préférence, l'inhibiteur de polymérisation bloque la réaction de polymérisation en complexant un catalyseur de polymérisation qui est également compris dans la composition.

Dans ce contexte, le catalyseur de polymérisation peut être formé par un ou plusieurs catalyseurs métalliques par exemple de type Dibutyltindilaureate (DBTL), ou Dioctyltindilaureate (DOTL). Cependant, il est préférable d'utiliser un ou plusieurs catalyseurs organométalliques qui peuvent être à base d'étain, d'aluminium, de zirconium, de cobalt, de bismuth, ou de zinc. En effet, les catalyseurs organométalliques permettent une réaction plus rapide que les catalyseurs métalliques précités, ils permettent d'assurer une polymérisation complète en bout de ligne de fabrication.

L'inhibiteur de polymérisation est formé par un agent chélatant, qui est adapté à réaliser un chélate du catalyseur de polymérisation. Dans cette optique, l'agent chélatant est introduit en excès par rapport au catalyseur de polymérisation.

Dans cet exemple, l'agent chélatant est un composé de type dicétone à tension de vapeur élevée 1.02 Kpa à 25°C. Cette caractéristique confère à l'agent chélatant une nature volatile. Un tel composé dicétone peut notamment être choisi dans la liste des composés suivants : éthyle acétoacétate, 3-éthyl-2,4-pentandione, 1,1,1-trifluoro-2,4-pentanedione, Triacéthyl méthane, 2,4-pentandione, 6-méthyl-2,4-heptadione,2,2,6,6-tétramethyl-3,5-heptadione,1-benzoyle acétone. L'inhibiteur de polymérisation peut être constitué par un de ces composés dicétoniques ou un mélange de ces composés.

En outre, solvanter la composition par un solvant polaire aprotique à tension de vapeur élevée, c'est-à-dire, un composé qui a une grande faculté d'évaporation lorsqu'il monte en température, permet d'éliminer facilement le solvant par évaporation. Ici, le solvant utilisé présente une tension de vapeur comprise entre 10 et 25 kPa à 25 °C.

De cette manière, il est possible d'obtenir une composition polymérique dans laquelle le pourcentage massique de solvant est inférieur 50 % par rapport la masse totale de la composition polymérique et de préférence il est possible d'utilisant un pourcentage massique de solvant inférieur à 35 % de la masse totale de la composition polymérique.

Le tableau 2 ci-dessous présente quatre exemples de composition selon la première variante de l'invention.

**[Tableau 2]**

| Composé | Exemple 1 | Exemple 2 | Exemple 3 | Exemple 4 |
|---|---|---|---|---|
| Nitrate d'argent en % massique | 3,5 | 1 | 2,5 | 4,5 |
| 1,2-butane diol en % massique | 5 | 6,5 | 5,7 | 6,1 |
| polyacrylate polyol en % massique | 20 | 26 | 22,8 | 24,4 |
| Oligomère d'isophorone diisocyanate en % massique | 26 | 35 | 31,5 | 33,3 |
| Dibutyltindilaureate en % massique | 0,2 | 0,5 | 0,4 | 1 |
| 2,4-pentandione en % massique | 1 | 1 | 1,2 | 3 |
| méthyl-pentyl-cétone en % massique | 44,3 | 30 | 35,9 | 27,7 |
| Total en % | 100 | 100 | 100 | 100 |

En outre, la seconde variante de la composition polymérique comporte deux précurseurs de polychlorure de vinyle. Selon l'invention la seconde variante de la composition polymérique comprend en pourcentages massiques par rapport à la masse totale :
∘ 1 à 6% de biocide ;
∘ 25 % à 45% comprend deux précurseurs de polychlorure de vinyle : une résine de chlorure de vinyle et un plastifiant polymérique, le ratio massique de chlorure de vinyle étant 3 :1 par rapport au plastifiant polymérique ;
∘ 50 % et 70 % d'au moins un solvant ;
∘ 1 à 5 % d'un agent de contrôle constitué par un stabilisant thermique de type sel de baryum.

De préférence, la seconde variante de la composition polymérique consiste en pourcentages massiques par rapport à la masse totale en :
∘ 1 à 6% de biocide ;
∘ 25 % à 45% comprend deux précurseurs de polychlorure de vinyle : une résine de chlorure de vinyle et un plastifiant polymérique, le ratio massique de chlorure de vinyle étant 3:1 par rapport au plastifiant polymérique ;
∘ 50 % et 70 % d'au moins un solvant ; et
∘ 1 à 5 % d'un agent de contrôle constitué par un stabilisant thermique de type sel de baryum.

La composition polymérique comprend de préférence une résine homopolymère de polychlorure de vinyle (PVC). La résine d'homopolymère de PVC possède une masse moléculaire moyenne comprise entre 150 000 et 250 000 g/mol. On peut aussi exprimer la masse moléculaire moyenne en K-Werts, ici la résine de PVC se présente sous la forme d'une poudre qui possède un K-Werts compris entre 60 et 90. Préférentiellement, la résine de PVC utilisée comprend un K-Werts compris entre 70 et 80.

De manière connue, PVC n'est pas un matériau irritant pour la peau. De fait, il est préférable de choisir une résine PVC qui possède un taux de monomères résiduels inférieur à 1 ppm. Ceci contribue à obtenir un film 1 antimicrobien dermocompatible.

De manière connue, le plastifiant améliore la résistance thermique et la stabilité à la lumière du PVC. En outre, le plastifiant permet d'augmenter l'élasticité du PVC à basse température. En pratique, le plastifiant est utilisé dans un ratio de 35 PCR, c'est-à-dire, 35 g de plastifiant pour 100 g de résine de chlorure de vinyle. Selon l'invention, le plastifiant est introduit dans la composition selon un ratio massique 1 pour 3 par rapport à la résine chlorure de vinyle.

De préférence, le plastifiant est un polymère polaire. Le plastifiant peut être choisi dans la famille des polyadipades ou des polysebaçates. De préférence, le plastifiant comporte une masse molaire moyenne comprise entre 2000 g/mole à 12 000 g/mole.

Le polyadipate de glycol répond à ces critères tout comme les produits commerciaux suivants : Palamoll 652®, Edenol 1233®, Santicizer 430®.

Cela contribue à assurer une bonne cohésion entre le plastifiant et la résine de chlorure de vinyle. Cela favorise également la solvatation du plastifiant dans un solvant polaire tel que les solvants dispersants décrits précédemment.

Pour les mêmes raisons que la résine de PVC, le plastifiant est choisi préférentiellement lorsqu'il comporte un taux de monomères résiduels inférieur à 1 ppm.

La seconde variante de la composition polymérique comporte préférentiellement un comportant un mélange d'un solvant dispersant et d'un solvant diluant. Le mélange de solvant comporte un pourcentage massique de solvant dispersant inférieur à 10% par rapport à la masse totale de solvant.

Le solvant diluant peut lui-même comprendre un mélange d'au moins un composé de nature aromatique avec au moins un composé de nature aliphatique. Ce mélange est de préférence réalisé selon un ratio 4 :1 de composé de nature aromatique.

De préférence, le solvant d'hydrocarbure aromatique est choisi aprotique. Ceci permet d'éviter toute réaction parasite avec la résine de PVC ou avec le plastifiant.

Le solvant d'hydrocarbure aromatique possède un nombre de carbone compris entre 7 et 10. De préférence, le solvant d'hydrocarbure aromatique peut être choisi parmi la liste des composés suivants : toluène, xylène, m-xylène, p-xylène, éthylbenzène, mésitylène, durène, ou un mélange de ces composés.

Selon l'invention, il est possible d'utiliser un solvant d'hydrocarbure aliphatique qui possède une formule chimique comprenant 6 et 13 carbones. Un solvant d'hydrocarbures aliphatiques dont la formule chimique possède entre 6 et 13 carbones ou un mélange de ces hydrocarbures aliphatiques.

Le solvant d'hydrocarbures aliphatiques peut être choisie parmi la liste de ces composés suivants : hexane, heptane, octane, nonane, décane, undécane, dodécane, tridécane, cyclohexène, cyclopentane, cyclohexane, cycloheptane, cyclooctane, cyclononane, méthyl-cyclohexane, cubane, ou un mélange de ces composés.

Il est également possible d'utiliser une coupe pétrolière de type Solvesso® 100, Hlsol®100 comme solvant d'hydrocarbures aliphatiques.

Dans cette seconde variante de l'invention, le stabilisant thermique de type sel de baryum contribue à donner à éviter que la résine de PVC ne se détériore lorsque la composition polymérique est exposée à une source de chaleur pour éliminer le solvant.

Le sel de baryum peut être choisi parmi la liste des composés commerciaux suivants : Baerostab® UBZ 674 X RF, Baerostab® UBZ 660-4 RF, Mark® 4821, Mark® 4864, Mark® RFD, LASTAB® DC 1595.

Le tableau3 ci-dessous présente quatre exemples de composition selon la seconde variante de l'invention.

**[Tableau 3]**

| Composé | Exemple 5 | Exemple 6 | Exemple 7 | Exemple 8 |
|---|---|---|---|---|
| Nitrate d'argent en % massique | 1,5 | 2 | 3 | 3,5 |
| Homopolymère PVC en % massique | 30 | 24 | 33 | 27 |
| Polyadipate de glycol en % massique | 10 | 8 | 11 | 9 |
| méthyl-butyl-cétone en % massique | 2,825 | 3,15 | 2,6 | 2,95 |
| p-xylène en % massique | 42,94 | 47,88 | 39,52 | 44,84 |
| heptane en % massique | 10,735 | 11,97 | 9,88 | 11,21 |
| Sel de baryum | 2 | 3 | 1 | 1,5 |
| Total en % | 100 | 100 | 100 | 100 |

L'invention concerne également un procédé de fabrication du film 1 antimicrobien qui utilise la composition polymérique selon l'invention.

Comme nous l'avons indiqué, le film 1 antimicrobien est coulé dans sa masse par une enduction de ladite composition polymérique à l'état liquide. Bien que le film 1 antimicrobien selon l'invention soit monobloc, il comporte au moins deux couches 2, 3, 6 polymérique comme nous l'avons décrit.

Le choix de la technique d'enduction pour former les couches 2, 3, 6 du film 1 antimicrobien permet de travailler à plus basse température que dans le cadre d'une formation d'un film polymérique par extrusion. Selon l'invention, la composition polymérique est portée à une température comprise entre 80°C et 200°C, alors que la technique d'extrusion applique une température supérieure à la température de fusion du matériau extrudé, soit 150°C pour le PVC et 250°C pour le polyuréthane. Néanmoins en pratique lors d'un procédé d'extrusion, les matériaux sont souvent soumis à des températures supérieures à 200°C voir supérieures à 250°C. De fait, la composition présente moins de contraintes en ce qui concerne la sensibilité thermique des composés comme le biocide.

En pratique, ladite composition polymérique est coulée sur un support solide d'un four de dix mètres de long. Or, il est difficile de réaliser une couche polymérique qui possède une épaisseur supérieure à 70 µm sur ce type de matériel. Dans ce contexte, la demanderesse à opté pour un procédé de fabrication d'un film 1 antimicrobien qui comporte au moins deux couches polymériques 2, 3, 6 superposées. Par superposées, il doit être compris que les couches polymériques 2, 3, 6 sont coulées les unes sur les autres.

Selon ce principe, le procédé de fabrication du film 1 antimicrobien selon l'invention comprend une étape de préparation d'une composition polymérique décrite précédemment qui intègre un biocide. Les différents composés de ladite composition polymérique sont de préférence mélangés sous agitation vigoureuse. La composition polymérique est mélangée jusqu'à obtention d'une solution à l'état liquide homogène et transparente.

Le procédé de fabrication comporte une étape de formation d'une première couche 2, 3 du film 1 à l'état cohésif. Cette étape comporte une opération d'enduction de ladite composition polymérique à l'état liquide sur un support solide. Dans cet exemple le support solide est plan et directement intégré dans un four industriel qui peut mesurer 10 mètres de long sur 2 mètres de large et au-delà de ces dimensions. L'opération d'enduction est accompagnée d'une opération d'étalement de la composition polymérique de manière à former une couche lisse dont l'épaisseur est comprise entre 40 µm et 120 µm. En effet, en fonction du pourcentage massique du solvant par rapport à la masse totale de composition polymérique, une couche polymérique 2, 3, 6 sèche peut perdre 20 à 50 % de l'épaisseur de la couche mouillée (chargée en solvant) qui a été étalée.

L'étape de formation de la première couche implique également une opération de séchage et/ou de polymérisation de ladite composition polymérique enduite sur le support solide afin d'obtenir une première couche polymérique à l'état cohésif. Cette opération de séchage est effectuée par application d'une température comprise entre 70°C et 200°C pendant une durée inférieure à 10 minutes.

On parle de polymérisation lorsque la composition polymérique comprend des précurseurs de polyuréthane telle que décrit dans la première variante de composition polymérique. Dans ce cas, la polymérisation démarre suite à l'évaporation de l'inhibiteur de polymérisation qui débute dès la montée en température de la composition polymérique étalée sur un support solide ou sur une couche polymérique à l'état cohésif. En pratique, le solvant utilisé présente une tension de vapeur plus importante et une température d'ébullition plus faible que l'inhibiteur de polymérisation, il est donc probable que le solvant soit évaporé avant le début de la réaction de polymérisation.

Par la suite, la polymérisation du polyuréthane est thermo catalysée par la chaleur appliquée à la composition polymérique.

Suite à l'opération de séchage, la première couche 2, 3 se trouve à l'état cohésif et repose directement sur le support du four. Une deuxième couche polymérique 2, 3, 6 est ensuite coulée à la surface de la première couche 2, 3.

Dans ce contexte, le procédé de fabrication comporte une étape de formation d'une deuxième couche 2, 3 du film 1 à l'état cohésif. Plus précisément, cette étape comprend une opération d'enduction de ladite composition polymérique sur une couche polymérique 2, 3, 6 à l'état cohésif déjà formée et reposant directement ou indirectement sur le support.

Une fois la deuxième enduction de la composition polymérique opérée, une opération de séchage et/ou de polymérisation est opérée afin que ladite composition polymérique enduite passe de l'état liquide à l'état cohésif. La deuxième couche polymérique 2, 3 est solidarisée à la première couche polymérique 2, 3 par thermocollage lors de la montée en température du four.

Le film 1 antimicrobien obtenu est monobloc et comprend deux couches 2, 3 intégrant un biocide. L'épaisseur du film 1 antimicrobien est comprise entre 50 µm et 300 µm.

Comme nous l'avons évoqué précédemment, il est difficile d'obtenir une couche polymérique supérieure 75 µm par induction dans les conditions matérielles décrites. Ainsi, lorsqu'on souhaite obtenir un film 1 antimicrobien dont l'épaisseur est supérieure à 140 µm, on réalise une troisième enduction d'une composition polymérique.

Pour des raisons économiques et comme le biocide ne migre pas du cœur de la couche polymérique vers sa surface, la troisième enduction vise à fournir un corps central qui n'est pas chargé en biocide comme illustré à la figure 2.

A cet effet le procédé de fabrication une étape de formation d'un corps central du film 1 qui est constitué par une couche polymérique 6. Ici, l'étape de formation du corps central est intercalée entre les étapes de formation de la première et de la deuxième couche 2, 3 comportant du biocide.

Selon l'invention, l'étape de formation du corps central comporte une opération de préparation d'une deuxième composition polymérique incluant un matériau thermoplastique élastomère ou des précurseurs de ce matériaux.

La composition polymérique du corps central selon l'invention comprend en pourcentages massiques par rapport à la masse totale :
∘ 25 à 70 % d'au moins un solvant,
∘ 25 à 70 % d'au moins un précurseur de polyuréthane ou d'au moins un précurseur de polychlorure de vinyle,
∘ 1 à 5 % d'au moins un agent de contrôle.

La composition polymérique du corps central selon l'invention consiste en pourcentages massiques par rapport à la masse totale en :
∘ 25 à 70 % d'au moins un solvant,
∘ 25 à 70 % d'au moins un précurseur de polyuréthane ou d'au moins un précurseur de polychlorure de vinyle,
∘ 1 à 5 % d'au moins un agent de contrôle.

La composition du corps central est également mélangée jusqu'à obtention d'un solution homogène et transparente à l'état liquide.

Il est à noter qu'il est possible de faire varier les natures ou les proportions des précurseurs de polyuréthane ou de polychlorure de vinyle tel que décrit précédemment pour la composition polymérique intégrant le biocide. Ceci permet de faire varier les propriétés mécaniques du corps central telles que l'élasticité.

L'étape de formation du corps central comporte une opération d'enduction de la composition polymérique du corps central sur la première couche 2, 3 à l'état cohésif qui repose sur le support du four.

-L'étape de formation du corps central se termine par une opération de séchage et/ou de polymérisation afin de faire passer la deuxième composition polymérique du corps central de l'état liquide à l'état cohésif, formant ainsi une couche polymérique 6 constituant le corps central.

Le corps central est bien entendu solidaire de la couche 2, 3 à l'état cohésif sur laquelle il a été coulé. Selon cette configuration, le corps central repose indirectement sur le support du four.

Selon ce mode de réalisation, la deuxième couche 2, 3 chargée en biocide est coulée sur le corps central à l'état cohésif.

Il est à noter qu'en fonction de l'épaisseur que l'on souhaite donner au corps central, il est possible d'opérer plusieurs opérations de coulage superposées les unes des autres. A titre indicatif, si l'on souhaite obtenir un corps central de 140 µm d'épaisseur, l'on peut réaliser deux coulages de 70 µm d'épaisseur.

Avant chaque opération d'enduction, le procédé de fabrication peut également comprendre une opération de filtration de la composition polymérique à l'état liquide. Ceci permet d'éliminer toutes particules, agrégats ou poussières présents dans la composition polymérique qui pourraient être préjudiciables à la qualité de la couche polymérique qui doit être parfaitement transparente et sans défaut. Cette opération de filtration la composition polymérique peut être effectuée par filtration sur une toile possédant une porosité comprise entre 20 µm et 80 µm.

Par ailleurs chaque opération d'enduction est associée à une opération d'étalement de la composition polymérique à la surface du support solide plan ou d'une couche polymérique à l'état cohésif. Cette opération d'étalement est effectuée à la barre Mayer. De manière connue, la barre Mayer est choisi en fonction de l'indice de son filetage. Ainsi, la quantité de la composition polymérique déposée sur le support est déterminée en fonction, de l'épaisseur de la couche polymérique que l'on souhaite on obtenir et de l'indice de la barre Mayer.

La composition polymérique perd en général entre 20 % et 50% de son épaisseur lorsqu'elle passe de l'état liquide à l'état cohésif. De fait pour obtenir une couche polymérique 2, 6 comprise entre 30 µm et 60 µm d'épaisseur, en pratique, la composition polymérique est étalée à l'état liquide selon une épaisseur comprise 40 µm et 120 µm.

Il est également possible d'effectuer l'enduction et l'étalement à lame d'air, au couteau sur cylindre, au couteau flottant, cylindre sur cylindre, trois ou quatre cylindres en direct ou reverse, la barre Mayer, la barre coma ou des méthodes habituellement utilisées par les imprimeurs telles que la flexographie, l'héliographie, etc.

Lorsque la fabrication du film 1 antibactérien est terminée, le film 1 est refroidi avant d'être enroulé sous la forme d'une bobine de 2 000 m linéaires.

Lorsque le film 1 antimicrobien est destiné à être utilisé dans le cadre d'un bloc opératoire, le procédé de fabrication et le conditionnement du film 1 antimicrobien peuvent se dérouler dans un local dont l'atmosphère est contrôlée telle qu'une chambre blanche.

La demanderesse a mené une étude expérimentale afin de s'assurer de l'activité virucide du film 1 antimicrobien à l'encontre du coronavirus humain SARS-CoV-2.

Cette étude se base le protocole de la norme ISO 21702.

Une première étude a été menée sur un film antimicrobien conforme de l'invention et possédant une teneur massique de 2% en masse par rapport à la masse totale de la couche qui l'intègre. En particulier, l'étude a été mené en utilisant du sel de phosphate d'argent, commercialisé sous le nom « silver phosphate glass® »,

Après une exposition de 60 minutes d'une quantité déterminé coronavirus humain SARS-CoV-2 en présence de cellules de culture à la surface de la couche chargée en agent biocide du film antimicrobien, cette première étude a montré l'inefficacité du film comportant une teneur massique en biocide inférieure à 10% à l'encontre du coronavirus humain SARS-CoV-2.

Une seconde étude a été menée sur un film antimicrobien 1 conforme de l'invention comprenant une teneur massique en biocide de 20% en masse par rapport à la masse totale de la couche qui intègre le biocide. En particulier, l'étude a été mené en utilisant du sel de phosphate d'argent, commercialisé sous le nom « silver phosphate glass® ».

Après une exposition de 60 minutes d'une quantité déterminée de coronavirus humain SARS-CoV-2 en présence de cellules de culture à la surface du film antimicrobien, une diminution de la quantité du coronavirus humain SARS-CoV-2 a été observée de 23.55% jusqu'à 76.04% avec une moyenne de 52.7%.

En conséquence, il est possible de corréler l'augmentation de la teneur en biocide dans la couche surface avec l'augmentation des cibles sur lesquels le film biocide possède une action et notamment l'efficacité du film antimicrobien 1 à l'encontre du SARS-CoV-2.]

## Revendications

1. Film (1) antimicrobien thermoplastique élastomère en polychlorure de vinyle ou en polyuréthane ou un mélange de ces matériaux, le film (1) antimicrobien étant coulé dans sa masse et intégrant un biocide, le biocide étant réparti directement dans la masse du film antimicrobien, **caractérisé en ce qu'**il comporte au moins deux couches (2 ,3, 6) superposées et thermo-soudées d'un matériau thermoplastique élastomère, au moins une des deux couches (2, 3) comporte le biocide selon une teneur massique déterminée par rapport à la masse de la couche (2, 3) qui intègre le biocide dans sa masse.

2. Film (1) antimicrobien selon la revendication 1, **caractérisé en ce que** les deux couches (2, 3) intègrent un biocide dans leur masse.

3. Film (1) antimicrobien selon la revendication 1, **caractérisé en ce qu'**il comporte au moins trois couches (2, 3, 6) superposées et thermo-soudées d'un matériau thermoplastique élastomère, le film (1) comportant un corps central recouvert supérieurement et inférieurement par deux couches (2, 3) externes, au moins l'une des deux couches (2, 3) externes intègre un biocide dans sa masse, de préférence, les deux couches (2, 3, 6) externes intègrent un biocide dans leur masse.

4. Film (1) antimicrobien selon l'une des revendications 1 à 3, **caractérisé en ce que** la teneur massique en biocide est comprise entre 0,1 et 30 % en masse par rapport à la masse totale d'une couche (2, 3) qui intègre le biocide, de préférence, la teneur massique en biocide est supérieure 10 % et inférieure à 30% en masse par rapport à la masse totale d'une couche (2, 3) qui intègre le biocide,

5. Film (1) antimicrobien selon l'une des revendications 1 à 4, le film (1) antimicrobien possédant en outre une épaisseur comprise entre 50 µm à 300 µm, de préférence le film antimicrobien possède une épaisseur comprise entre 100 µm à 200 µm.

6. Film (1) antimicrobien selon l'une des revendications 1 à 5, **caractérisé en ce qu'**il est étanche à l'air et à l'eau.

7. Film (1) antimicrobien selon l'une des revendications 1 à 6, **caractérisé en ce qu'**il possède une transparence d'au moins 70 % à la lumière visible, de préférence, il possède une transparence d'au moins 90 % à la lumière visible.

8. Film (1) antimicrobien selon l'une des revendications 1 à 7, **caractérisé en ce qu'**il comporte un allongement à la rupture d'au moins 150 %, de préférence il possède un allongement à la rupture d'au moins 180%.

9. Film (1) antimicrobien selon l'une des revendications 1 à 8, **caractérisé en ce qu'**il possède une résistance à la traction d'au moins 2 kN/m, de préférence, la résistance à la traction est d'au moins 4kN/m.

10. Film (1) antimicrobien selon l'une des revendications 1 à 9, **caractérisé en ce qu'**il est dermo-compatible.

11. Film (1) antimicrobien selon l'une des revendications 1 à 10, pour l'utilisation comme un drap médical ou comme une protection de matériel médical contre la transmission de micro-organismes infectieux.

12. Composition polymérique de préparation d'un film (1) antimicrobien selon l'une des revendications 1 à 11, **caractérisée en ce qu'**elle comprend en pourcentages massiques par rapport à la masse totale :
• 1 à 6 % d'un biocide,
• 25 à 70 % d'au moins un solvant,
• 25 à 70 % d'au moins un précurseur de polyuréthane ou d'au moins un précurseur de polychlorure de vinyle,
• 1 à 5 % d'au moins un agent de contrôle.

13. Composition polymérique selon la revendication 12, le biocide étant un composé aprotique stable thermiquement à des températures supérieures à 100°C, de préférence, le biocide est un composé stable thermiquement à des températures supérieures à 150°C.

14. Composition polymérique selon l'une des revendications 12 et 13, le biocide étant un sel d'argent ou un composé de type isothiazole.

15. Composition polymérique selon l'une des revendications 12 à 14, **caractérisée en ce que** le solvant comporte un solvant dispersant choisi parmi les composés suivants :
∘ un solvant polaire aprotique de type acétate choisi parmi les solvants suivants : l'acétate de méthyle, acétate d'éthyle, acétate de propyle, acétate de butyle, acétate de pentyle, acétate d'hexyle, acétate d'heptyle, acétate d'octyle ou un mélange de ces composés ; ou
∘ un solvant polaire aportique de type cétonique et choisi parmi les solvants suivants : di-méthyl-cétone, éthyl-méthyl-cétone, propyl-méthyl-cétone, butyl-méthyl-cétone, méthyl-butyl-cétone, méthyl-isobutyl-cetone, méthyl-pentyl-cétone, di-éthyl-cétone, éthyl-propyl-cétone, éthyl-butyl-cétone, éthyl-pentyl-cétone, di-propyl-cétone, butyl-propyl-cétone, pentyl-propyl-cétone, di-butyl-cétone, butyl-pentyl-cétone, ou un mélange de ces composés.

16. Composition polymérique selon l'une des revendications 12 à 15, en ce qu'elle comprend en pourcentages massiques par rapport à la masse totale :
• 1 à 6% de biocide ;
• 25 % à 45% comprend deux précurseurs de polychlorure de vinyle : une résine de chlorure de vinyle et un plastifiant polymérique, le ratio massique de chlorure de vinyle étant 1 :3 par rapport au plastifiant polymérique.
• 45 % à 70 % d'au moins un solvant ;
• 1 à 5 % d'un agent de contrôle constitué par un stabilisant thermique de type sel de baryum.

17. Composition polymérique selon la revendication 16, la résine de chlorure de vinyle étant un homopolymère de masse moléculaire moyenne comprise entre 150 000 g/mol et 250 000 g/mol.

18. Composition polymérique selon l'une des revendications 16 et 17, le plastifiant polymérique est de type polyadipate ou polysebaçate.

19. Composition polymérique selon l'une des revendications 16 à 18, le solvant comportant un mélange d'un solvant dispersant et d'un solvant diluant, le solvant diluant est également composé d'un mélange d'au moins un composé de nature aromatique avec au moins un composé de nature aliphatique selon un ratio 4 :1, le solvant diluant étant choisi parmi les composés suivants :
∘ Un solvant d'hydrocarbure aromatique aprotique possédant 7 à 10 carbones choisi parmi la liste des composés suivants :
toluène, xylène, m-xylène, p-xylène, éthylbenzène, mésitylène, durène, ou un mélange de ces composés ;
∘ Un solvant d'hydrocarbures aliphatiques dont la formule chimique possède entre 6 et 13 carbones ou un mélange de ces hydrocarbures aliphatiques.

20. Composition polymérique selon l'une des revendications 12 à 15, en ce qu'elle comprend en pourcentages massiques par rapport à la masse totale :
• 1 à 6% de biocide ;
• 10% à 35% d'un premier précurseur de polyuréthane comprenant un mélange d'un composé polyol à courtes chaines et d'un composé polyol à longues chaines selon un ratio du composé polyol à courtes chaines 1 :4 par rapport au composé polyol à longue chaines ;
• 15 % à 35% d'un second précurseur de polyuréthane comportant au moins un composé poly-isocyanate ;
• 25 % et 50 % d'au moins un solvant ;
• 1 à 5 % d'un agent de contrôle comprenant un inhibiteur de polymérisation.

21. Composition polymérique selon la revendication 20, l'agent de contrôle comportant en outre un catalyseur de polymérisation qui est chelaté par l'inhibiteur de polymérisation.

22. Procédé de fabrication d'un film antimicrobien l'une des revendications 1 à 11, **caractérisé en ce qu'**il comprend :
∘ une étape de préparation d'une composition polymérique selon l'une des revendications 12 à 21 qui intègre un biocide, la composition étant mélangée jusqu'à obtention d'une solution à l'état liquide homogène et transparente ;
∘ une étape de formation d'une première couche (2, 3) du film (1) à l'état cohésif qui comporte :
- une opération d'enduction de ladite composition polymérique à l'état liquide sur un support solide, et
- une opération de séchage et/ou de polymérisation de ladite composition polymérique enduite sur le support solide afin d'obtenir une première couche polymérique à l'état cohésif ;
∘ une étape de formation d'une deuxième couche (2, 3) du film (1) à l'état cohésif qui comporte :
- une opération d'enduction de ladite composition polymérique sur une couche polymérique (2, 3, 6) à l'état cohésif reposant directement ou indirectement sur le support, et
- une opération de séchage et/ou de polymérisation de ladite composition polymérique enduite à l'état liquide afin d'obtenir une deuxième couche polymérique à l'état cohésif permettant d'obtenir un film (1) antimicrobien monobloc comprenant, d'une part, deux couches (2, 3) intégrant un biocide, et d'autre part, une épaisseur comprise entre 50 µm et 300 µm,.

23. Procédé de fabrication selon la revendication 22, comprenant une étape de formation d'un corps central du film (1) qui est constitué par au moins une couche polymérique (6), l'étape de formation du corps central comportant :
- Une opération de préparation d'une deuxième composition polymérique incluant un matériau thermoplastique élastomère ou des précurseurs de ce matériaux, la deuxième composition étant mélangée jusqu'à obtention d'un solution homogène et transparente à l'état liquide,
- Une opération d'enduction de la deuxième composition polymérique sur la première couche (2, 3) à l'état cohésif,
- Une opération de séchage et/ou de polymérisation de la deuxième composition polymérique afin de la faire passer de l'état liquide à l'état cohésif formant ainsi une couche polymérique (6) constituant le corps central solidaire de la couche (2, 3) à l'état cohésif sur laquelle il a été enduit,
l'étape de formation du corps central est intercalée entre les étapes de formation de la première et de la deuxième couche (2, 3) comportant du biocide.

24. Procédé de fabrication selon l'une des revendications 22 et 23, chaque opération de séchage est effectuée par application d'une température comprise entre 70°C et 200°C pendant une durée inférieure à 10 minutes.
